# EUROPEAN PATENT APPLICATION

(11) **EP 3 711 606 A1**
(43) Date of publication of application: **23.09.2020**
(21) Application number: 19164460.8
(22) Date of filing: 21.03.2019
(51) Int. Cl.: A24F 47/00, A61M 11/04, A61M 15/06

(54) **AEROSOL DELIVERY SYSTEM**

(71) Applicant: NERUDIA LIMITED, Liverpool Merseyside L24 9HP (GB)
(72) Inventor: The designation of the inventor has not yet been filed
(74) Representative: Mewburn Ellis LLP

(57) **Abstract**

An aerosol delivery system (10) has a fluid transfer article (34) with a first region (34a) for holding an aerosol precursor and a second region (34b) to which the aerosol precursor is transferred from the first region. The second region includes a multiplicity of fibres which extend from the first region to an end of the fluid-transfer article which is at or proximate a heating surface of a heater (24) of the aerosol delivery system. The aerosol precursor passes along the fibres and interacts thermally with the heater to form an aerosol from said aerosol precursor. The first region may be formed from a porous polymer material. An air-flow pathway extends along the heating surface with that air-flow pathway passing among the fibres. When the user sucks or inhales through the aerosol delivery system, air-flows through the air-flow pathway along and the heating surface, so that aerosol passes from the heater and/or the ends of the fibres to the user in the air-flow.

## Description

### Field of the Invention

The present invention relates to an aerosol delivery system, and an aerosol-generation apparatus for an aerosol delivery system. The present invention preferably relates to an aerosol delivery system including a heater configured to heat an aerosol precursor to generate an aerosolised composition for inhalation by a user, and to an aerosol-generation apparatus therefor.

### Background

Pharmaceutical medicament, physiologically active substances and flavourings for example may be delivered to the human body by inhalation through the mouth and/or nose. Such material or substances may be delivered directly to the mucosa or mucous membrane lining the nasal and oral passages and/or the pulmonary system. For example, nicotine is consumed for therapeutic or recreational purposes and may be delivered to the body in a number of ways. Nicotine replacement therapies are aimed at people who wish to stop smoking and overcome their dependence on nicotine. Nicotine is delivered to the body in the form of aerosol delivery devices and systems, also known as smoking-substitute devices or nicotine delivery devices. Such devices may be non-powered or powered.

Devices or systems that are non-powered may comprise nicotine replacement therapy devices such as "inhalators", e.g. Nicorette® Inhalator. These generally have the appearance of a plastic cigarette and are used by people who crave the behaviour associated with consumption of combustible tobacco - the so-called hand-to-mouth aspect - of smoking tobacco. Inhalators generally allow nicotine-containing aerosol to be inhaled through an elongate tube in which a container containing a nicotine carrier, for example, a substrate, is located. An air stream caused by suction through the tube by the user carries nicotine vapours into the lungs of the user to satisfy a nicotine craving. The container may comprise a replaceable cartridge, which includes a cartridge housing and a passageway in the housing in which a nicotine reservoir is located. The reservoir holds a measured amount of nicotine in the form of the nicotine carrier. The measured amount of nicotine is an amount suitable for delivering a specific number of "doses". The form of the nicotine carrier is such as to allow nicotine vapour to be released into a fluid stream passing around or through the reservoir. This process is known as aerosolization and or atomization. Aerosolization is the process or act of converting a physical substance into the form of particles small and light enough to be carried on the air i.e. into an aerosol. Atomization is the process or act of separating or reducing a physical substance into fine particles and may include the generation of aerosols. The passageway generally has an opening at each end for communication with the exterior of the housing and for allowing the fluid stream through the passageway. A nicotine-impermeable barrier seals the reservoir from atmosphere. The barrier includes passageway barrier portions for sealing the passageway on both sides of the reservoir. These barrier portions are frangible so as to be penetrable for opening the passageway to atmosphere.

A device or a system that is powered can fall into two sub-categories. In both subcategories, such devices or systems may comprise electronic devices or systems that permit a user to simulate the act of smoking by producing an aerosol mist or vapour that is drawn into the lungs through the mouth and then exhaled. The electronic devices or systems typically cause the vaporization of a liquid containing nicotine and entrainment of the vapour into an airstream. Vaporization of an element or compound is a phase transition from the liquid phase to vapour i.e. evaporation or boiling. In use, the user experiences a similar satisfaction and physical sensation to those experienced from a traditional smoking or tobacco product, and exhales an aerosol mist or vapour of similar appearance to the smoke exhaled when using such traditional smoking or tobacco products.

A person of ordinary skill in the art will appreciate that devices or systems of the second, powered category as used herein include, but are not limited to, electronic nicotine delivery systems, electronic cigarettes, e-cigarettes, e-cigs, vaping cigarettes, pipes, cigars, cigarillos, vaporizers and devices of a similar nature that function to produce an aerosol mist or vapour that is inhaled by a user. Such nicotine delivery devices or systems of the second category incorporate a liquid reservoir element generally including a vaporizer or misting element such as a heating element or other suitable element, and are known, inter alia, as atomizers, cartomizers, or clearomizers. Some electronic cigarettes are disposable; others are reusable, with replaceable and refillable parts.

Aerosol delivery devices or systems in a first sub-category of the second, powered category generally use heat and/or ultrasonic agitation to vaporize a solution comprising nicotine and/or other flavouring, propylene glycol and/or glycerine-based base into an aerosol mist of vapour for inhalation.

Aerosol delivery devices or systems in a second sub-category of the second, powered category may typically comprise devices or systems in which tobacco is heated rather than combusted. During use, volatile compounds may be released from the tobacco by heat transfer from the heat source and entrained in air drawn through the aerosol delivery device or system. Direct contact between a heat source of the aerosol delivery device or system and the tobacco heats the tobacco to form an aerosol. As the aerosol containing the released compounds passes through the device, it cools and condenses to form an aerosol for inhalation by the user. In such devices or systems, heating, as opposed to burning, the tobacco may reduce the odour that can arise through combustion and pyrolytic degradation of tobacco.

Aerosol delivery devices or systems falling into the first sub-category of powered devices or systems may typically comprise a powered unit, comprising a heater element, which is arranged to heat a portion of a carrier that holds an aerosol precursor. The carrier comprises a substrate formed of a "wicking" material, which can absorb aerosol precursor liquid from a reservoir and hold the aerosol precursor liquid. Upon activation of the heater element, aerosol precursor liquid in the portion of the carrier in the vicinity of the heater element is vaporised and released from the carrier into an airstream flowing around the heater and carrier. Released aerosol precursor is entrained into the airstream to be borne by the airstream to an outlet of the device or system, from where it can be inhaled by a user.

The heater element is typically a resistive coil heater, which is wrapped around a portion of the carrier and is usually located in the liquid reservoir of the device or system. Consequently, the surface of the heater may always be in contact with the aerosol precursor liquid, and long-term exposure may result in the degradation of either or both of the liquid and heater. Furthermore, residues may build up upon the surface of the heater element, which may result in undesirable toxicants being inhaled by the user. Furthermore, as the level of liquid in the reservoir diminishes through use, regions of the heater element may become exposed and overheat.

The present invention has been devised in light of the above considerations.

### Summary of the Invention

At its most general, the present invention proposes that an aerosol-generation apparatus has a fluid-transfer article which holds aerosol precursor and which has a multiplicity of fibres which act as a wick for the aerosol precursor. The fibres extend to, or proximate, a heating surface of a heater of the aerosol-generation apparatus. An air-flow pathway can then be defined along the heating surface, passing through the multiplicity of fibres.

Thus, aerosol precursor will be drawn into the spaces between the fibres. When the heating surface is active, aerosol precursor at or adjacent that heating surface will be vaporized, to form vapour and/or a vapour and aerosol mixture. Air flow along the air-flow pathway will cause that vapour or mixture to pass along the heating surface and out from the spaces between the fibres. This will have the effect of drawing more fluid along the fibres towards the heating surface, for the process to continue.

Thus, according to the present invention, there may be provided an aerosol-generation apparatus comprising a heater and a fluid-transfer article, the fluid-transfer article comprising a first region for holding an aerosol precursor and for transferring said aerosol precursor to a second region of said fluid-transfer article, said second region being formed from a multiplicity of fibres, each of said multiplicity of fibres extending from said first region to an end of said fluid-transfer article facing said heater, said multiplicity of fibres terminating at, or proximate a heating surface of said heater, there being an air-flow pathway along said heating surface, which air-flow pathway passes among said multiplicity of fibres.

Optionally, the first region of the fluid-transfer article comprises an empty tank for the receipt and storage of said aerosol precursor.

Alternatively, the first region of the fluid-transfer article is made of a porous material such as a porous polymer material. That porous material may hold aerosol precursor, and pass the aerosol precursor to the spaces between the ends of the fibres remote from the heating surface. For example, the porous material may end at a transfer surface, with the ends of the fibres in contact with that transfer surface.

The porous polymer material may comprise Polyetherimide (PEI) and/or Polyether ether ketone (PEEK) and/or Polytetrafluoroethylene (PTFE) and/or Polyimide (PI) and/or Polyethersulphone (PES) and/or Ultra-High Molecular Weight Polyethylene (UHMWPE) and/or Polypropylene (PP) and/or Polyethylene Terephthalate (PET).

The fibres are preferably generally parallel as they extend from the first region of the fluid-transfer article towards the heater.

The aerosol-generation apparatus may form part of an aerosol delivery system which has a carrier and include a housing containing the fluid-transfer apparatus. There may then be a further housing containing the heater, with the housing and the further housing being separable. The further housing may have an inlet and outlet, with the air-flow pathway extending to the inlet and outlet.

The invention includes the combination of the aspects and preferred features described except where such a combination is clearly impermissible or expressly avoided.

### Summary of the Figures

So that the invention may be understood, and so that further aspects and features thereof may be appreciated, embodiments illustrating the principles of the invention will now be discussed in further detail with reference to the accompanying figures, in which:
**Figure 1****.** is a perspective view illustration of a system for aerosol delivery according to one or more embodiments of the present invention;
**Figure 2****.** is a cross-section side view illustration of part of an apparatus of the system for aerosol delivery of Figure 1;
**Figure 3****.** is a cross-section side view illustration of the system and apparatus for aerosol delivery of Figure 1;
**Figure 4****.** is a perspective view illustration of an aerosol carrier for use in the system for aerosol delivery according to one or more embodiments of the present invention;
**Figure 5****.** is a cross-section side view of elements of an aerosol carrier and a part of an apparatus of the system for aerosol delivery according to one or more embodiments of the present invention;
**Figure 6****.** is a cross-section side view of elements of an aerosol carrier and a part of an apparatus of the system for aerosol delivery according to one or more embodiments of the present invention, in an alternative configuration from that of Figure 5;
**Figure 7****.** is a cross-section side view of aerosol carrier according to one or more embodiments of the present invention;
**Figure 8****.** is a perspective cross-section side view of the aerosol carrier of Figure 7, and;
**Figure 9****.** is an exploded perspective view illustration of a kit-of-parts for assembling the system according to one or more embodiments of the present invention.

### Detailed Description of the Invention

Aspects and embodiments of the present invention will now be discussed with reference to the accompanying figures. Further aspects and embodiments will be apparent to those skilled in the art. All documents mentioned in this text are incorporated herein by reference.

In general outline, one or more embodiments in accordance with the present invention may provide a system for aerosol delivery in which an aerosol carrier may be inserted into a receptacle (e.g. a "heating chamber") of an apparatus for initiating and maintaining release of an aerosol from the aerosol carrier. Another end, or another end portion, of the aerosol carrier may protrude from the apparatus and can be inserted into the mouth of a user for the inhalation of aerosol released from the aerosol carrier cartridge during operation of the apparatus.

Hereinafter, and for convenience only, "system for aerosol delivery" shall be referred to as "aerosol delivery system".

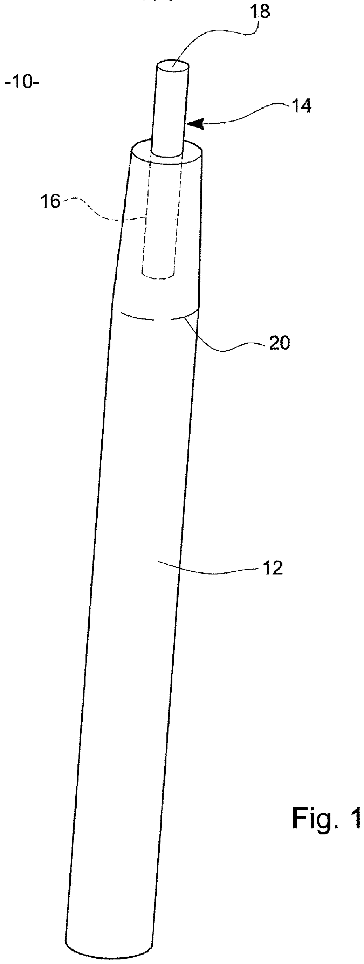

Referring now to Figure 1, there is illustrated a perspective view of an aerosol delivery system 10 comprising an aerosol generation apparatus 12 operative to initiate and maintain release of aerosol from a fluid-transfer article in an aerosol carrier 14. In the arrangement of Figure 1, the aerosol carrier 14 is shown with a first end 16 thereof and a portion of the length of the aerosol carrier 14 located within a receptacle of the apparatus 12. A remaining portion of the aerosol carrier 14 extends out of the receptacle. This remaining portion of the aerosol carrier 14, terminating at a second end 18 of the aerosol carrier, is configured for insertion into a user's mouth. A vapour and/or aerosol is produced when a heater (not shown in Figure 1) of the apparatus 12 heats a fluid-transfer article in the aerosol carrier 14 to release a vapour and/or an aerosol, and this can be delivered to the user, when the user sucks or inhales, via a fluid passage in communication with an outlet of the aerosol carrier 14 from the fluid-transfer article to the second end 18.

The device 12 also comprises air-intake apertures 20 in the housing of the apparatus 12 to provide a passage for air to be drawn into the interior of the apparatus 12 (when the user sucks or inhales) for delivery to the first end 16 of the aerosol carrier 14, so that the air can be drawn through an activation region of a fluid-transfer article located within a housing of the aerosol carrier cartridge 14 during use. Optionally, these apertures may be perforations in the housing of the apparatus 12.

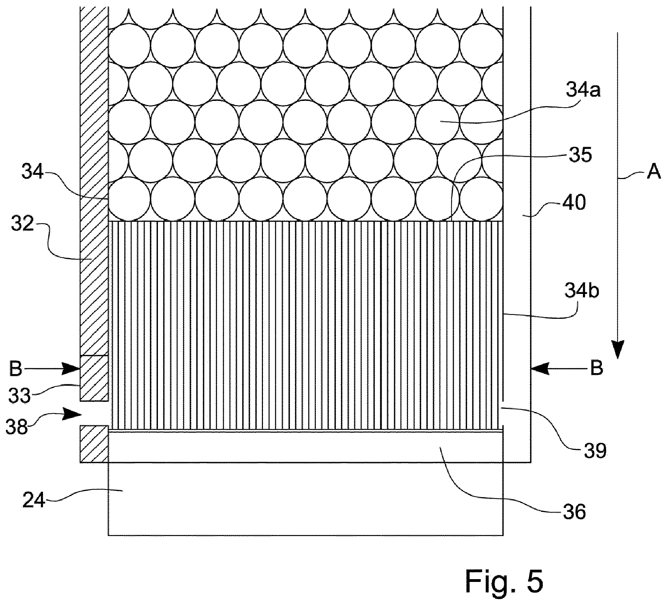

A fluid-transfer article (not shown in Figure 1, but described hereinafter with reference to Figs. 5 to 8 is located within a housing of the aerosol carrier 14. The fluid-transfer article contains an aerosol precursor material, which may include at least one of: nicotine; a nicotine precursor material; a nicotine compound; and one or more flavourings. The fluid-transfer article is located within the housing of the aerosol carrier 14 to allow air drawn into the aerosol carrier 14 at, or proximal, the first end 16 to flow at an activation region of the fluid-transfer article. As air passes through the activation region of the fluid-transfer article, an aerosol may be entrained in the air stream from a substrate forming the fluid-transfer article, e.g. via diffusion from the substrate to the air stream and/or via vaporisation of the aerosol precursor material and release from the fluid-transfer article under heating.

The fluid-transfer article 34 may comprise a first region of a porous material where pores of the porous material hold, contain, carry, or bear the aerosol precursor material, and a second region formed of a multiplicity of fibres. In particular, the porous material of the fluid-transfer article is a porous polymer material such as, for example, a sintered material. Particular examples of material suitable for the fluid-transfer article include: Polyetherimide (PEI); Polytetrafluoroethylene (PTFE); Polyether ether ketone (PEEK); Polyimide (PI); Polyethersulphone (PES); and Ultra-High Molecular Weight Polyethylene. Other suitable materials may comprise, for example, BioVyonTM (by Porvair Filtration Group Ltd) and materials available from Porex®. Further optionally, a substrate forming the fluid-transfer article may comprise Polypropylene (PP) or Polyethylene Terephthalate (PET). All such materials may be described as heat resistant polymeric wicking material in the context of the present invention.

The aerosol carrier 14 is removable from the apparatus 12 so that it may be disposed of when expired. After removal of a used aerosol carrier 14, a replacement aerosol carrier 14 can be inserted into the apparatus 12 to replace the used aerosol carrier 14.

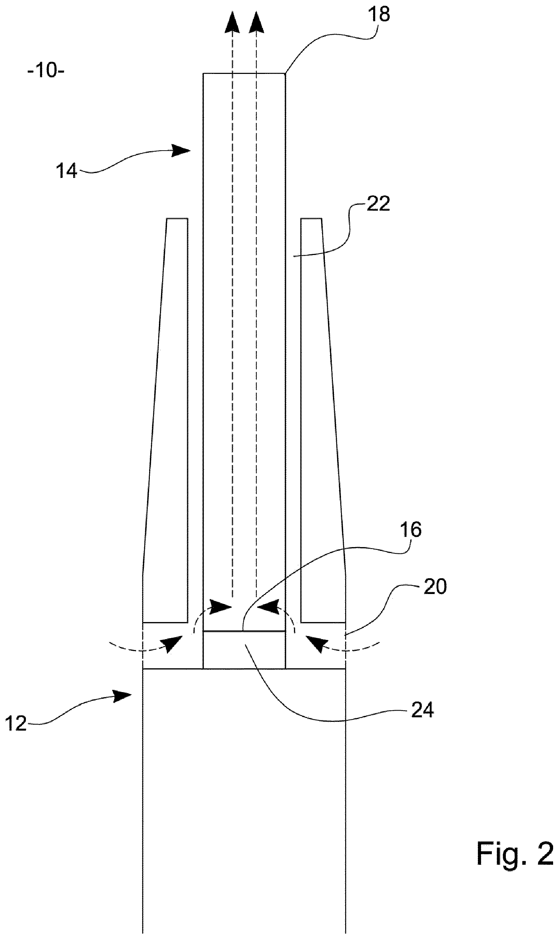

Figure 2 is a cross-sectional side view illustration of a part of apparatus 12 of the aerosol delivery system 10. The apparatus 12 comprises a receptacle 22 in which is located a portion of the aerosol carrier 14. In one or more optional arrangements, the receptacle 22 may enclose the aerosol carrier 14. The apparatus 12 also comprise a heater 24, which opposes an activation region of the fluid-transfer article (not shown in Figure 2) of the aerosol carrier 14 when an aerosol carrier 14 is located within the receptacle 22.

Air flows into the apparatus 12 (in particular, into a closed end of the receptacle 22) via air-intake apertures 20. From the closed end of the receptacle 22, the air is drawn into the aerosol carrier 14 (under the action of the user inhaling or sucking on the second end 18) and expelled at the second end 18. As the air flows into the aerosol carrier 14, it passes an end of the fluid-transfer article or across that end. Heat from the heater 24, which opposes that end of the fluid-transfer article (the activation region), causes vaporisation of aerosol precursor material at the end of the fluid-transfer article and an aerosol is created in the air flow. Thus, through the application of heat in the region of the end of the fluid-transfer article, an aerosol is released, or liberated, from the fluid-transfer article, and is drawn from the material of the aerosol carrier unit by the air flow and is transported in the air flow to via outlet conduits (not shown in Figure 2) in the housing of the aerosol carrier 14 to the second end 18. The direction of air flow is illustrated by arrows in Figure 2.

To achieve release of the captive aerosol from the fluid-transfer article, the fluid-transfer article of the aerosol carrier 14 is heated by the heater 24. As a user sucks or inhales on second end 18 of the aerosol carrier 14, the aerosol released from the fluid-transfer article and entrained in the air is drawn through the outlet conduits (not shown) in the housing of the aerosol carrier 14 towards the second end 18 and onwards into the user's mouth.

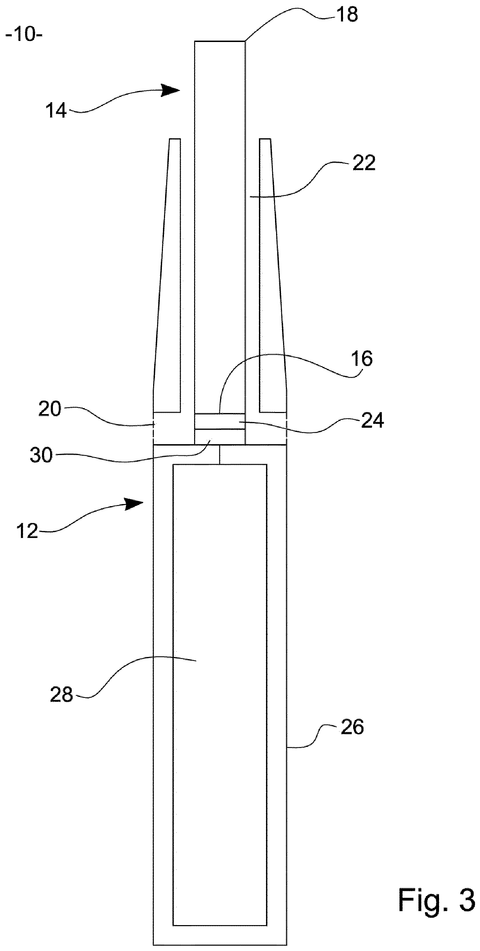

Turning now to Figure 3, a cross-sectional side view of the aerosol delivery system 10 is schematically illustrated showing the features described above in relation to Figures 1 and 2 in more detail. As can be seen, apparatus 12 comprises a housing 26, in which are located the receptacle 22 and heater 24. The housing 26 also contains control circuitry (not shown) operative by a user, or upon detection of air and/or vapour being drawn into the device 12 through air-intake apertures 20, i.e. when the user sucks or inhales. Additionally, the housing 26 comprises an electrical energy supply 28, for example a battery. Optionally, the battery comprises a rechargeable lithium ion battery. The housing 26 also comprises a coupling 30 for electrically (and optionally mechanically) coupling the electrical energy supply 28 to control circuitry (not shown) for powering and controlling operation of the heater 24.

Responsive to activation of the control circuitry of apparatus 12, the heater 24 heats the fluid-transfer article (not shown in Figure 3) of aerosol carrier 14. This heating process initiates (and, through continued operation, maintains) release of vapour and/or an aerosol from the end of the fluid-transfer article adjacent the heater. The vapour and/or aerosol formed as a result of the heating process is entrained into a stream of air being drawn across the heating surface of the heater (as the user sucks or inhales). The stream of air with the entrained vapour and/or aerosol passes through the aerosol carrier 14 via outlet conduits (not shown) and exits the aerosol carrier 14 at second end 18 for delivery to the user. This process is briefly described above in relation to Figure 2, where arrows schematically denote the flow of the air stream into the device 12 and through the aerosol carrier 14, and the flow of the air stream with the entrained vapour and/or aerosol through the aerosol carrier cartridge 14.

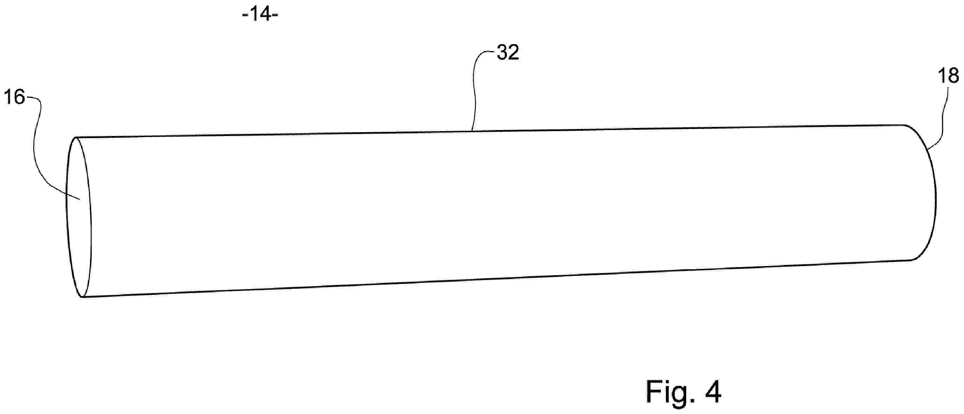

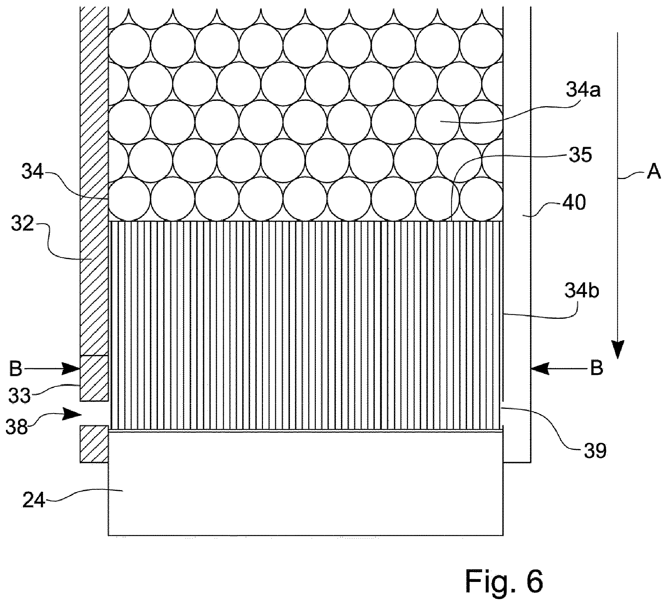

Figures 4 to 6 schematically illustrate the aerosol carrier 14 in more detail (and, in Figures 5 and 6, features within the receptacle in more detail). Figure 4 illustrates an exterior of the aerosol carrier 14, Figure 5 illustrates internal components of the aerosol carrier 14 in one optional configuration, and Fig. 6 illustrates internal components of the aerosol carrier 14 in another optional configuration.

Fig. 4 illustrates the exterior of the aerosol carrier 14, which comprises a housing 32 for housing said fluid- transfer article (not shown). The particular housing 32 illustrated in Figure 4 comprises a tubular member, which may be generally cylindrical in form, and which is configured to be received within the receptacle of the apparatus. First end 16 of the aerosol carrier 14 is for location to oppose the heater of the apparatus, and second end 18 (and the region adjacent the second end 18) is configured for insertion into a user's mouth.

Figure 5 illustrates some internal components of the aerosol carrier 14 and of the heater 24 of apparatus 12, in in one embodiment of the invention.

As described above, the aerosol carrier 14 comprises a fluid-transfer article 34. Optionally, there may be a conduction element 36 (as shown in Figure 5), being part of the heater 24. In one or more arrangements, the aerosol carrier 14 is located within the receptacle of the apparatus such that an end of the fluid-transfer article opposes and is adjacent the heater 24 of the apparatus and receives heat directly from the heater 24 of the apparatus. When aerosol carrier 14 is located within the receptacle of the apparatus such that the adjacent end of the fluid-transfer article is located to oppose the heater of the apparatus, the conduction element 36 is disposed between the rest of the heater 24 and the end of the fluid-transfer article. Heat may be transferred to the end of the fluid-transfer article via conduction through conduction element 36 (i.e. application of heat to the activation surface is indirect).

Further components not shown in Figure 5 comprise: an inlet conduit, via which air can be drawn into the aerosol carrier 14; an outlet conduit, via which an air stream entrained with aerosol can be drawn from the aerosol carrier 14; a filter element; and a reservoir for storing aerosol precursor material and for providing the aerosol precursor material to the fluid-transfer article 34.

In Figure 5, the aerosol carrier is shown as comprising the fluid-transfer article 34 located within housing 32. The fluid transfer article 34 comprises a first region 34a holding an aerosol precursor. In one or more arrangements, the first region of 34a of the fluid transfer article 34 comprises a reservoir for holding the aerosol precursor. The first region 34a can be the sole reservoir of the aerosol carrier 14, or it can be arranged in fluid communication with a separate reservoir, where aerosol precursor is stored for supply to the first region 34a. In the particular arrangement illustrated, the material forming the first region of 34a comprises a porous structure, whose pore diameter size may vary between one end of the first region 34a and another end of the first region 34a. For example, the pore diameter size may decrease from a first end remote from heater 24 (the upper end is as shown in the figure) to a second end. This configuration of pores having a decreasing diameter size can provide a wicking effect, which can serve to draw fluid through the first region 34a, towards heater 24. As mentioned above, the porous polymer material may be a sintered material. Particular examples of material suitable for the fluid-transfer article include: Polyetherimide (PEI); Polytetrafluoroethylene (PTFE); Polyether ether ketone (PEEK); Polyimide (PI); Polyethersulphone (PES); and Ultra-High Molecular Weight Polyethylene. Other suitable materials may comprise, for example, BioVyonTM (by Porvair Filtration Group Ltd) and materials available from Porex®. Further optionally, a substrate forming the fluid-transfer article may comprise Polypropylene (PP) or Polyethylene Terephthalate (PET).

Alternatively, the first region 34a may be a simple liquid reservoir in the form of an empty tank for the receipt and storage of liquid aerosol precursor, rather than porous material for holding the aerosol precursor.

The fluid transfer article 34 also comprises a second region 34b. In arrangements in which the first region 34a is formed from material having a porous structure, aerosol precursor is drawn from the first region 34a to the second region 34b by the wicking effect of the material. Thus, the first region 34a is configured to transfer the aerosol precursor to the second region 34b of the article 34.

The second region 34b is formed of a multiplicity of fibres. The fibres may be arranged in a generally parallel manner, with small spaces therebetween. The fibres extend from an end of the first region 34a, which end will be referred to as a transfer surface, towards the heater 24. In Figure 5, where the optional conduction element 36 is present, the fibres forming the second region 34b extend to that conduction element 36. However, it is possible that for them to terminate just short of the conduction element 36 if desired. The fibres forming the second region may be glass fibres, Kevlar fibres, etc.

Aerosol precursor within the first region 34a will pass into the spaces between the fibres of the second region 34b, and be drawn along the fibres by capillary action. Aerosol precursor will thus pass to end of the second region 34b proximate to the conduction element 36, which region of the fibres acts as an activation region for the aerosol precursor. When the heater 24 is active, heat will be transferred via that conduction element 36 to the aerosol precursor at the adjacent end of the second region 34b which forms the activation region (i.e. at the ends of the fibres furthest from the transfer surface 35) cause that aerosol precursor to vaporise.

Figure 5 also illustrates an opening 38 in a further housing 33, which opening 38 is in communication with the air-intake apertures 20. A further opening 39 communicates with a duct 40 within the housing 32, which duct 40 communicates with the second end 18.

The further housing 33 supports the heater 24 (and optional conduction element 36 if present). The housing 32 and the further housing 33 are separable, e.g. along the line B-B in Figure 5, to allow the housing 32, and hence the fluid-transfer article 34, to be removed from the rest of the apparatus. Since the aerosol precursor in the fluid-transfer article will be consumed as the user uses the apparatus, it will be necessary periodically to replace it. This can be done by removing the housing 32 from the rest of the apparatus, and refilling the aerosol precursor, or replacing the housing 32 with another in which the fluid-transfer article is full of aerosol precursor. The further housing 33 may be integral with the housing 26 containing the electrical energy supply 28.

There is thus a fluid-flow path for air (hereinafter referred to as an air-flow pathway) between openings 38 and 39, linking the apertures 20 and the second end 18 of the aerosol carrier. When the user sucks or inhales, air is drawn along the air-flow pathway, along the surface of the conduction element 36, and between the ends of the fibres forming in the second region 34b (and between the ends of he fibres and the conduction element 36 if the fibres terminate just short of the conduction element 36).

Aerosol precursor which has reached the ends of the fibres of the second region 34b and has been heated and transferred by the conduction element 36 will pass into the air flowing in the air-flow pathway between the openings 38 and 39. The vapour or mixture passes, as the user sucks and inhales, to the second end 18. This has the effect of removing aerosol precursor from the ends of the fibres proximate the conduction element 36. This will have the effect of drawing further aerosol precursor down the fibres from the transfer surface 35. There may also be a low-pressure effect due to the movement of the air along the air-flow pathway, which draws fluid along the fibres to the conduction element 36. There may be a small gap between the ends of the fibres forming the second region of 34b and the conduction element 36, with the air flow is through that space. However, it is preferable that the fibres of the second region 34b extend to the conduction element 36, to ensure maximum heat transfer to the aerosol precursor. In either case, the air-flow pathway will pass around the ends of the fibres, in the small spaces between the fibres, to enable vapour and/or aerosol and vapour mixture to enter the air flow. The spacing between each of the fibres is desirably chosen so as to facilitate that air flow, but also to create a capillary effect which will transfer aerosol precursor from the transfer surface 35.

As noted above, the conduction element 36 may be absent in some arrangements.

The conduction element 36, if present, may comprise a thin film of thermally conductive material, such as, for example, a metal foil (for example, aluminium, brass, copper, gold, steel, silver, or an alloy comprising anyone of the foregoing together with thermally conductive plastics and/or ceramics).

In the illustrative examples of Figure 5, the first region 34a of the fluid-transfer article 34 is located at an "upstream" end of the fluid-transfer article 34 and the second region 34b is located at a downstream" end of the fluid-transfer article 34. That is, aerosol precursor is wicked, or is drawn, from the "upstream" end of the fluid-transfer article 34 to the "downstream" end of the fluid-transfer article 34 (as denoted by arrow A in Figure 5).

As mentioned above, the conduction element 36 need not be present. Figure 6 illustrates an embodiment corresponding to that of Figure 5, but without such a conduction element 36. The arrangement of Figure 6 is otherwise similar to that of Figure 5, and corresponding parts are indicated by the same reference numerals.

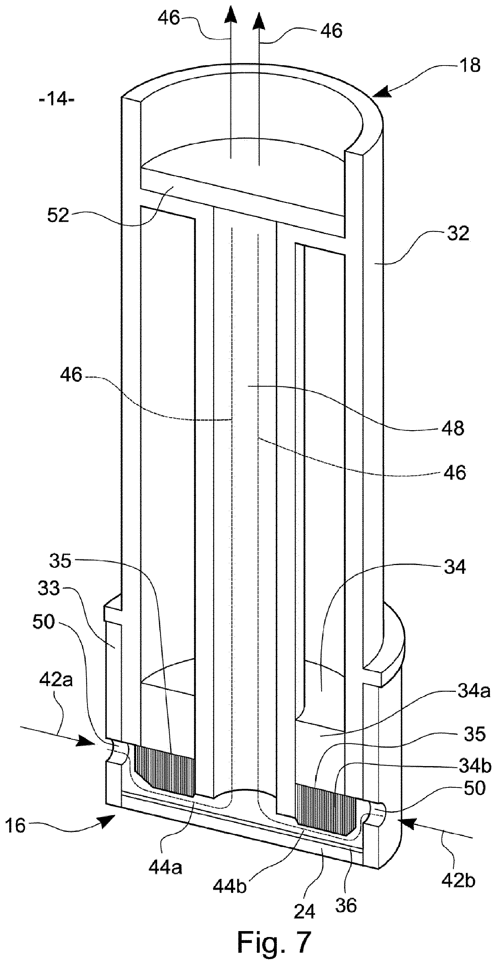

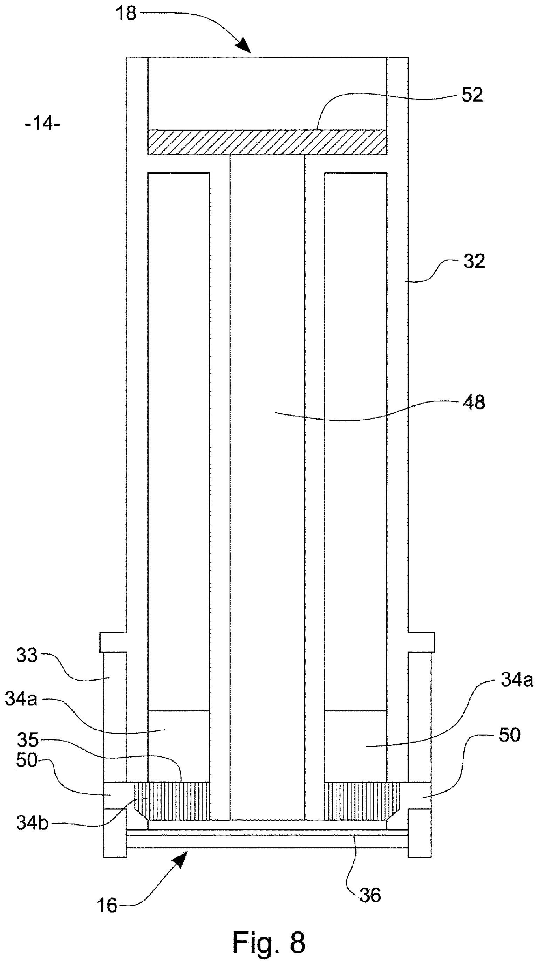

In the arrangements shown in Figures 5 and 6, the apertures 38, 39 are on opposite sides of the housing 32. Figures 7 and 8 shows an alternative configuration, in which the fluid-transfer article is annular, and the second part 34b is then in the form of annular diaphragm. Note that, in Figures 7 and 8, the ends of the fibres forming the second part 34b are illustrated terminating just short of the conduction element 36. This is a possible arrangement, but it is also possible that the fibres extend to the conduction surface 36, as in the arrangements of Figures 5 and 6. The small space between the ends of the fibres and the conduction surface 36 in Figures 7 and 8 is shown to enable the airflow in the apparatus to be illustrated. Thus, Figures 7 and 8 illustrate an aerosol carrier 14 according to one or more possible arrangements in more detail. Figure 7 is a cross-section side view illustration of the aerosol carrier 14 and Figure 8 is a perspective cross-section side view illustration of the aerosol carrier 14.

As can be seen from Figures 7 and 8, the aerosol carrier 14 is generally tubular in form. The aerosol carrier 14 comprises housing 32, which defines the external walls of the aerosol carrier 14 and which defines therein a chamber in which are disposed the fluid-transfer article 34 (adjacent the first end 16 of the aerosol carrier 14) and internal walls defining the fluid communication pathway 48. Fluid communication pathway 48 defines a fluid pathway for an outgoing air stream from the channels 40 to the second end 18 of the aerosol carrier 14. In the examples illustrated in Figures 7 and 8, the fluid-transfer article 34 is an annular shaped element located around the fluid communication pathway 48. As in the arrangements of Figure 5 and 6, the heater 24 (and optional conduction element 36 if present) are mounted in a further housing 33, which further housing 33 is separable from the housing 32 containing the fluid-transfer article.

In walls of the further housing 33, there are provided inlet apertures 50 to provide a fluid communication pathway for an incoming air stream to reach the fluid-transfer article 34, and particularly the air-flow pathway defined across the surface of the conduction element 36 (or across the surface of the heater 24), and passing among the ends of the fibres forming the second region 34b of the aerosol-transfer article 34.

In the illustrated example of Figures 7 and 8, the aerosol carrier 14 further comprises a filter element 52. The filter element 52 is located across the fluid communication pathway 48 such that an outgoing air stream passing through the fluid communication pathway 48 passes through the filter element 52.

With reference to Figure 8, when a user sucks on a mouthpiece of the apparatus (or on the second end 18 of the aerosol carrier 14, if configured as a mouthpiece), air is drawn into the carrier through inlet apertures 50 extending through walls in the housing 32 of the aerosol carrier 14.

An incoming air stream 42a from a first side of the aerosol carrier 14 is directed to a first side of the second part 34b of the fluid-transfer article 34 (e.g. via a gas communication pathway within the housing of the carrier). An incoming air stream 42b from a second side of the aerosol carrier 14 is directed to a second side of the second part 34a of the fluid-transfer article 34 (e.g. via a gas communication pathway within the housing of the carrier). When the incoming air stream 42a from the first side of the aerosol carrier 14 reaches the first side of the second part 34b, the incoming air stream 42a from the first side of the aerosol carrier 14 flows around the ends of the fibres of the second part 34b across the conduction element 36 (or across the heater 24). Likewise, when the incoming air stream 42b from the second side of the aerosol carrier 14 reaches the second side of the second part 34a, the incoming air stream 42b from the second side of the aerosol carrier 14 flows around the ends of the fibres of the second part 34a across the conduction element 36 (or across heater 24). The air streams from each side are denoted by dashed lines 44a and 44b in Figure 8. As these air streams 44a and 44b flow, aerosol precursor at the ends of the fibres or on the conduction element 36 (or on the heater 24) is entrained in air streams 44a and 44b.

In use, the heater 24 of the apparatus 12 to raise a temperature of the conduction element 36 to a sufficient temperature to release, or liberate, captive substances (i.e. the aerosol precursor) to form a vapour and/or aerosol, which is drawn downstream. As the air streams 44a and 44b continue their passages, more released aerosol precursor is entrained within the air streams 44a and 44b. When the air streams 44a and 44b entrained with aerosol precursor meet at a mouth of the outlet fluid communication pathway 48, they enter the outlet fluid communication pathway 48 and continue until they pass through filter element 52 and exit outlet fluid communication pathway 48, either as a single outgoing air stream, or as separate outgoing air streams 46 (as shown). The outgoing air streams 46 are directed to an outlet, from where it can be inhaled by the user directly (if the second end 18 of the aerosol capsule 14 is configured as a mouthpiece), or via a mouthpiece. The outgoing air streams 46 entrained with aerosol precursor are directed to the outlet (e.g. via a gas communication pathway within the housing of the carrier).

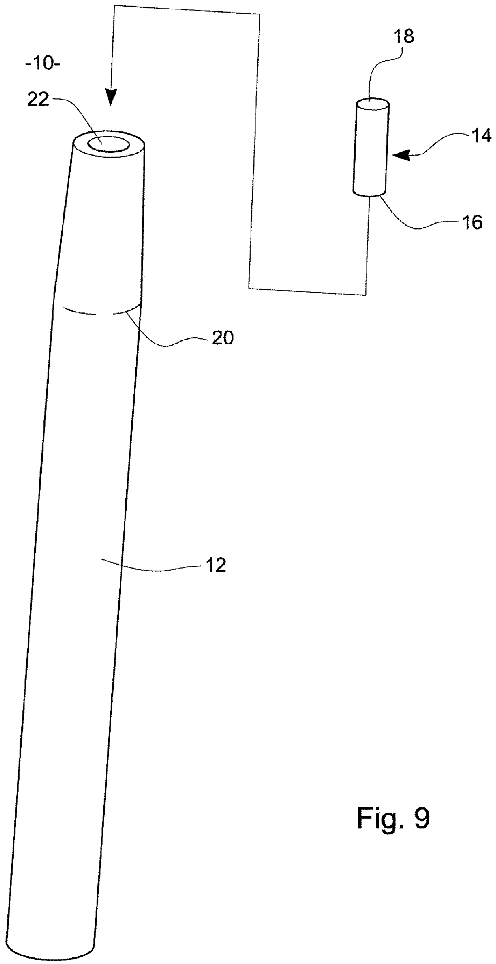

Figure 9 is an exploded perspective view illustration of a kit-of-parts for assembling an aerosol delivery system 10.

As will be appreciated, in the arrangements described above, the fluid-transfer article 34 is provided within a housing 32 of the aerosol carrier 14. In such arrangements, the housing of the carrier 14 serves to protect the aerosol precursor-containing fluid-transfer article 34, whilst also allowing the carrier 14 to be handled by a user without his/her fingers coming into contact with the aerosol precursor liquid retained therein.

The features disclosed in the foregoing description, or in the following claims, or in the accompanying drawings, expressed in their specific forms or in terms of a means for performing the disclosed function, or a method or process for obtaining the disclosed results, as appropriate, may, separately, or in any combination of such features, be utilised for realising the invention in diverse forms thereof.

While the invention has been described in conjunction with the exemplary embodiments described above, many equivalent modifications and variations will be apparent to those skilled in the art when given this disclosure. Accordingly, the exemplary embodiments of the invention set forth above are considered to be illustrative and not limiting. Various changes to the described embodiments may be made without departing from the spirit and scope of the invention.

For the avoidance of any doubt, any theoretical explanations provided herein are provided for the purposes of improving the understanding of a reader. The inventors do not wish to be bound by any of these theoretical explanations.

Any section headings used herein are for organizational purposes only and are not to be construed as limiting the subject matter described.

Throughout this specification, including the claims which follow, unless the context requires otherwise, the words "have", "comprise", and "include", and variations such as "having", "comprises", "comprising", and "including" will be understood to imply the inclusion of a stated integer or step or group of integers or steps but not the exclusion of any other integer or step or group of integers or steps.

It must be noted that, as used in the specification and the appended claims, the singular forms "a," "an," and "the" include plural referents unless the context clearly dictates otherwise. Ranges may be expressed herein as from "about" one particular value, and/or to "about" another particular value. When such a range is expressed, another embodiment includes from the one particular value and/or to the other particular value. Similarly, when values are expressed as approximations, by the use of the antecedent "about," it will be understood that the particular value forms another embodiment. The term "about" in relation to a numerical value is optional and means, for example, +/- 10%.

The words "preferred" and "preferably" are used herein refer to embodiments of the invention that may provide certain benefits under some circumstances. It is to be appreciated, however, that other embodiments may also be preferred under the same or different circumstances. The recitation of one or more preferred embodiments therefore does not mean or imply that other embodiments are not useful, and is not intended to exclude other embodiments from the scope of the disclosure, or from the scope of the claims.

## Claims

1. An aerosol-generation apparatus comprising a heater and a fluid-transfer article, the fluid-transfer article comprising a first region for holding an aerosol precursor and for transferring said aerosol precursor to a second region of said fluid-transfer article, said second region being formed from a multiplicity of fibres, each of said multiplicity of fibres extending from said first region to an end of said fluid-transfer article facing said heater, said multiplicity of fibres terminating at, or proximate a heating surface of said heater, there being an air-flow pathway along said heating surface, which air-flow pathway passes among said multiplicity of fibres.

2. An aerosol-generation apparatus according to claim 1, wherein the multiplicity of fibres are generally parallel.

3. An aerosol-generation apparatus according to claim 1 or claim 2, wherein the first region of said fluid-transfer article comprises an empty tank for the receipt and storage of said aerosol precursor.

4. An aerosol-generation apparatus according to claim 1 or claim 2, wherein the first region of said fluid-transfer article is formed from porous polymer material.

5. An aerosol-generation apparatus according to claim 4, wherein said porous polymer material comprises Polyetherimide (PEI).

6. An aerosol-generation apparatus according to claim 4, wherein said porous polymer material comprises Polyether ether ketone (PEEK).

7. An aerosol-generation apparatus according to claim 4, wherein said porous polymer material comprises Polytetrafluoroethylene (PTFE).

8. An aerosol-generation apparatus according to claim 4, wherein said porous polymer material comprises Polyimide (PI).

9. An aerosol-generation apparatus according to claim 4, wherein said polymeric wicking material comprises Polyethersylphone (PES).

10. An aerosol-generation apparatus article according to claim 4, wherein said porous polymer material comprises Ultra-High Molecular Weight Polyethylene (UHMWPE)

11. An aerosol-generation apparatus according to claim 4, wherein said porous polymer material comprises Polypropylene (PP).

12. An aerosol-generation apparatus article according to claim 4, wherein said porous polymer material comprises Polyethylene Terephthalate (PET).

13. An aerosol delivery system comprising an aerosol-generation apparatus according to any one of the claims 1 to 12 and a carrier comprising a housing containing said fluid-transfer article.

14. An aerosol-delivery system according to claim 13, further including a further housing containing said heater, said housing and said further housing being separable.

15. An aerosol delivery system according to claim 14, wherein said further housing has an inlet and an outlet, said air-flow pathway extending between said inlet and said outlet.
